# EUROPEAN PATENT APPLICATION

(11) **EP 4 292 512 A1**
(43) Date of publication of application: **20.12.2023**
(21) Application number: 22178885.4
(22) Date of filing: 14.06.2022
(51) Int. Cl.: A61B 1/24, A61C 17/22, A46B 15/00, G06T 7/00

(54) **A DEVICE FOR USE IN THE MOUTH FOR CAPTURING IMAGES OF ORAL TISSUE**

(71) Applicant: Koninklijke Philips N.V., 5656 AG Eindhoven (NL)
(72) Inventor: HIWALE, Sujitkumar, Eindhoven (NL); GERHARDT, Lutz Christian, Eindhoven (NL); JOHNSON, Mark Thomas, Eindhoven (NL); KOOIJMAN, Gerben, Eindhoven (NL); PERRONE, Antonio Luigi, Eindhoven (NL)
(74) Representative: Philips Intellectual Property & Standards

(57) **Abstract**

A device (10, 60) is for use in the mouth, for implementing a primary function. An optical imaging system (18, 64) is mounted on a body of the device for capturing images of oral tissue in contact with the imaging system. Contact of an image collection surface of the imaging system with oral tissue to be imaged is detected, and one or more images are stored during said contact, for further analysis or transmission.

## Description

### FIELD OF THE INVENTION

The invention relates to devices for use in the mouth, such as oral care devices, or baby teats or soothers. It relates in particular to such devices which can capture images for the purpose of screening oral symptoms for example to enable oral conditions to be diagnosed.

### BACKGROUND OF THE INVENTION

Assessment of surface properties of the oral structures can provide useful information on the oral and systemic health status of an individual. The different oral and systemic diseases lead to characteristic changes in morphological and histological features of the oral cavity structures.

Changes to the tongue and oral mucosa (e.g., hyperplasia, herpes, ulcers) are common in autoimmune diseases and in hematological, endocrine, and neoplastic processes. The prevalence of oral mucosa lesions has been found to be age-dependent with an overall prevalence of about 12 %. Similar prevalence has been also reported for tongue lesions or diseases. For example, in an Indian study, the prevalence of tongue lesions was found to be about 14 %.

There is a wide range of very different conditions that bring modifications to the oral cavity and its soft tissues. Autoimmune diseases may manifest as oral ulcerations and lesions on the tongue. Hematological disorders such as anemia, red blood cell disorders and nutritional deficiencies may present with gingival bleeding or tongue changes such as glossitis. Oral changes associated with endocrine illness are variable and depend on the underlying condition. Neoplastic changes include metastatic lesions to the bony and soft tissues of the oral cavity. Patients with chronic diseases such as gastroesophageal reflux and eating disorders may present with dental erosions that cause oral pain or halitosis.

Moreover, many newborns and babies suffer from oral thrush, a white lip or tongue coating due to fungal infection which is difficult to be distinguished from milk residues (milk tongue). Approximately 5 - 10 % of infants develop oral thrush, and mainly in the first 10 weeks of life, although the problem can appear later as well. Oral thrush is also a known problem in "fragile" infants and/or those born prematurely.

Oral manifestations of local and systemic diseases develop rather slowly. In the absence of a regular follow-up with a dentist, there is a high chance that these manifestations are either missed or diagnosed late in the disease progression cycle.

It is known to use imaging systems to analyze oral tissue appearance to detect oral health conditions. Conventional imaging-based screening tools such as camera-based applications (e.g. using a fixed camera or a mobile phone camera) or intra-oral scanners are likely to be of a limited use in this scenario due to known poor compliance with such tools. They are usually used sporadically and also typically when it is too late (the progression of the disease is well advanced). The requirement of additional time, equipment, and cost are important factors in poor compliance with these methods.

The chronic nature of disease progression also makes these methods less reliable as they are mostly used on ad hoc basis. In addition, most camera-based apps and intra-oral scanners operate in a non-contact imaging mode; therefore, they cannot be used to image the tongue or other oral tissue during the normal oral hygiene routine - such as tooth brushing - as this requires the imaging device to be in contact with the oral tissue while imaging.

Thus, existing methods for assessment of oral structures (i.e., visual inspection/palpation, camera-based methods using smartphones or intraoral scanners) have certain disadvantages associated with them, including:
extra hassle for the user or professional as extra scanning time is needed and imaging is not included in the workflow of using a personal care device;
subjectivity in assessment;
inability to study morphological features of oral the surfaces as part of an oral hygiene routine;
poor user compliance due to need of dedicated time, equipment and accessories for assessment;
the need for separate (often lengthy) scanning procedures (e.g. up to 2 minutes) by usually a dental professional.

It would be of interest to have an easy method to assess surface properties of oral structures on a regular basis without need of any additional user intervention.

### SUMMARY OF THE INVENTION

The invention is defined by the claims.

According to examples in accordance with an aspect of the invention, there is provided a device for use in the mouth, for implementing a primary function, wherein the device comprises:
a body;
an optical imaging system mounted at the body for capturing images of oral tissue in contact with the optical imaging system, wherein the optical imaging system has an image collection surface and the capturing of images comprises a secondary function of the device; and
a processor for controlling the collection and/or storage of images by the optical imaging system,
wherein the device is configured to detect contact of the image collection surface with oral tissue to be imaged, and
wherein the processor is configured to store one or more images during said contact for further analysis or transmission.

The invention provides a device with a primary in-mouth function (e.g. a tooth treatment function such as brushing, flossing or irrigating etc., or a baby feeding or pacifying function) and which provides a secondary imaging function. The images can be collected while performing the primary function (e.g., during brushing) with no extra hassle for user to take images e.g., after brushing with a different device such as a smart phone.

Other examples of the primary function include dental instrument functions, whereby the dental instrument will make random contact with oral tissue (e.g., the back-side of a mirror that touches the cheek).

Whilst a user is busy with an oral routine (brushing) or infant feeding or soothing, the surfaces of the oral device which are not used to perform the primary in-mouth function tend to make random contact with (other adjacent) tissues in the oral cavity (such as the cheek, lip, tongue, palate). The invention is also based on efficiently exploiting this side feature to obtain insights into oral health.

In the case of a treatment device, the imaging is for example for non-treatment surfaces of the device. These non-treatment surfaces may also make contact with other tissues in the oral cavity. For example the back of a toothbrush platen will make contact with the tongue or check mucosal tissue (e.g. when brushing lingual or buccal molars, lingual or buccal incisors). The platen is particularly in contact with the tongue or cheeks when a person brushes with a closed mouth, but frequent random contact also occurs in persons who brush with an open mouth. This contact can thus be used for contact-triggered imaging and image storage of tissue structures adjacent and opposite to the treatment/brushing area. Similarly, in the case of a baby feeding or pacifying function, the oral tissue of the baby may also make random contact with the surface of the device during use.

By detecting contact, which is when the images are suitable for storage and subsequent analysis, only the suitable images are stored. The images may be stored locally or they may be stored only temporarily for transmission to a remote device where they are then stored for further analysis. The contact detection is thus to identify images of interest, and they are processed accordingly, and differently to images not of interest. For example, they may be processed differently to images not of interest (e.g. stored in memory vs. not retained in memory, or transmitted vs. not transmitted), or else images not of interest may not be captured at all.

The further analysis is typically used to identify oral manifestations or pathologies. This further analysis may be in real time or offline.

The optical imaging system for example comprises a fixed focus camera and/or a contact microscope, for contact imaging. Direct contact imaging provides higher image magnification and resolution so the images are potentially of greater diagnostic value. By storing/processing/transmitting only images during contact times, there is a reduction of required data volume. Microscope optics are particularly suitable for contact imaging, with a fixed focus distance.

The device may comprise an apparatus for implementing the primary function to a first oral tissue, and wherein the optical imaging system is for implementing the secondary function to a different second oral tissue.

This "apparatus" for example comprises toothbrush bristles, or an oral irrigator nozzle, or a powered toothbrush drivetrain, or a head of a dental tool, etc. Thus, the nature of the apparatus for implementing the primary function depends on the main function of the device.

The second oral tissue may be of a different type to the first (e.g. the first may be the teeth and the second may be the tongue or cheek) or they may be of the same type but at different locations. The primary function is for example not intended to be applied to the second oral tissue. The optical imaging system for example makes random contact with the second oral tissue during the implementation of the primary function.

The processor is for example configured to apply a first contact threshold to detect when contact is made and to store the one or more images in response to said contact. It may also apply a second contact threshold to cease storing images.

The two thresholds are used to determine the appropriate time period during which images should be captured and stored.

In one example, the processor is configured to detect contact based on detection of an image focus. For fixed focus contact imaging, the image will only be in focus when there is contact with the imaging surface. Thus, images can be captured, and only when contact is detected will suitable images for further processing be stored.

In another example, the device comprises a driven portion (e.g. a driven head), and the processor is configured to detect contact based on analysis of an image movement. When there is contact between the driven portion and oral tissue, the tissue may move in synchronism with the head, so that image movement reduces during contact.

In another example, the device comprises a driven portion and a load sensor for sensing a drive load of the driven portion, and the processor is configured to detect contact from the drive load. The driven load will change e.g. increase when an external contact is present and this may be detected from the drive parameters, e.g. current or voltage. Thus the load sensor may be for sensing drive parameters, hence detecting the actual load indirectly.

In another example, the device comprises a force sensor for sensing a force applied to the device, and the processor is configured to detect contact from the sensed force. This force sensor thus functions as a contact sensor. This may for example apply to the force applied to a pacifier or feeding teat.

In another example, the device comprises a proximity sensor, wherein the processor is configured to detect contact from an output of the proximity sensor. The proximity sensor may be optical or it may be a capacitive sensor.

In another example, the device comprises a location sensing system, wherein the processor is configured to detect contact from an output of the location sensing system. The location sensing system is for example based on an inertia monitoring unit which tracks the movement of the device. Location sensing of hand held devices is well known, and some locations will correspond to tissue contact.

In one set of examples, the device comprises an oral care or treatment device and the primary function is to clean or treat oral tissue. The oral care function may comprise flossing, brushing, or irrigating. The oral treatment function may comprise RF or light based treatment, or it may comprise a whitening function of a whitening mouthpiece or a light delivery mouthpiece.

In one example, the device comprises a powered toothbrush and the primary function is tooth brushing.

In the case of a toothbrush, it may comprise platen having a front side from which oral cleaning elements (e.g., bristles) extend, and the image collection surface is on a back side or lateral side of the platen. Thus, the imaging is based on a surface away from the bristles. This reduces the issue of toothpaste foam hindering the imaging. As explained above, there are then two different types of oral tissue surface for the two functions of the device.

In another set of examples, the device comprises a baby pacifier, wherein the primary function is to provide a simulated nipple to pacify a baby.

In another set of examples, the device comprises a teat for a baby feeding bottle, wherein the primary function is to provide a simulated nipple for baby feeding.

The invention also provides a method of capturing images of an internal surface of the oral cavity using a device which is for implementing a primary function and comprises a body, and an optical imaging system with an image collection surface, mounted at the body for capturing images of oral tissue in contact with the optical imaging system, the method comprising:
detecting contact of the image collection surface with oral tissue to be imaged; and
storing one or more images during said contact for further analysis or transmission,
wherein the capturing of images comprises a secondary function of the device.

The method may comprise applying a first contact threshold to detect when contact is made and to store the one or more images in response to said contact, and applying a second contact threshold to cease storing images.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a power toothbrush incorporating a fixed focus camera;
Figure 2 shows a toothbrush head to show a preferred camera placement;
Figure 3 shows a method of capturing images of an internal surface of the oral cavity;
Figure 4 shows a first example of a teat which incorporates a camera sensor; and
Figure 5 shows a second example of a teat which incorporates a camera sensor.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for use in the mouth, for implementing a primary function. A fixed focus camera is mounted on a body of the device for capturing images of oral tissue in contact with the camera as a secondary function. Contact of an image collection surface of the camera with oral tissue to be imaged is detected, and one or more images are stored during said contact, for further analysis.

The device may be any device which is used to perform a primary function which involves application in the mouth, e.g. for brushing the teeth, treating the teeth, treating the gums, flossing or irrigating. It also applies to baby bottles or pacifiers and to dental equipment (mirrors, tools etc.).

The invention will be explained with reference to a power toothbrush, although the same principles may be applied to other devices as outlined above.

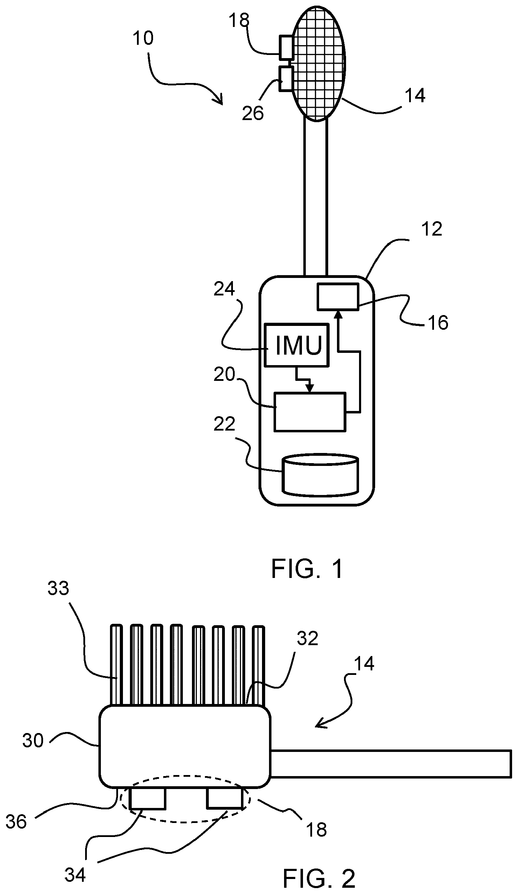

Figure 1 shows a power toothbrush 10 comprising a handle 12 and a toothbrush head 14. The head is driven by a drive motor 16. The head is the part that enters the mouth and hence has a body which is for use in the mouth. The primary function of the toothbrush is of course for brushing the teeth.

An optical imaging system, preferably a fixed focus camera 18 for contact imaging, is mounted on the head body for capturing images of oral tissue in contact with an image collection surface of the camera 18. Images are captured of tissue in contact with that image collection surface. The image collection surface is for example a focusing lens. The capturing of images comprises a secondary function of the toothbrush. In particular, this secondary function can be performed while the primary function is being performed. Thus, in the case of a toothbrush, image capture can take place while the user is performing a tooth brushing session.

The optical imaging system may be recessed in an opening (or an opening with a window), and the desired contact for imaging may then be with the opening or window.

A processor 20 is used for controlling the camera, namely controlling the collection of images (i.e. activation of the camera) and/or the storage of images in a memory 22.

The processor 20 detects contact of the image collection surface with oral tissue to be imaged and then stores one or more images during that contact for further analysis.

As will be explained below, the contact detection may make use of image analysis or it may use separate sensing. Figure 1 shows an optional inertial monitoring unit 24 which may be used to determine when contact is made, and an optional separate contact sensor 26 for the same purpose. These options will be discussed below.

If image analysis is used for contact sensing (hence not needing any additional sensors), images are captured for analysis periodically. When contact is detected, the images are used differently, for example they may be captured at a greater sampling frequency or with greater resolution, and they are stored and subjected to analysis for diagnostic purposes (in real time) or stored for subsequent analysis for diagnostic purposes (offline). Thus, the contact sensing may be part of a low power mode and the image capture and storage may be part of a high power mode.

If separate contact sensing is used, images may only be captured when contact is detected, so the camera can be in a sleep or idle mode at other times. Thus, when contact is detected, the images are captured and also stored for future further processing, or they are stored and further processed in real time.

In both cases, the further processing is used to identify oral and systemic diseases which result in characteristic changes in morphological and histological features of the oral cavity structures. This image processing is known and will not be described in further detail.

A first set of examples uses analysis of the camera images to generate a skin contact sensing signal.

With close focus (microscopic) imaging, the depth of field will be quite small, so only when there is contact with the oral tissue, such as with the tongue, will the image appear sharp. Determination of the focus by image analysis is well known, for example using contrast-based focus detection. The obtained images are then processed to determine their properties.

If the obtained images are of a certain predefined quality (e.g., light intensity, sharpness, contrast) then it is considered that there is contact with the tissue and storing of the images is initiated.

Another image-based method which can be used in the case of a driven device (like a power toothbrush) is to assess if the image is moving under influence of the vibration. Image motion will be considerably reduced when the toothbrush head makes some contact, or it may even stop in some cases, e.g., if the tongue papillae can follow the e.g. 200Hz motion. Thus, images are stored only when there is a reduction in the image motion with respect to the free vibration frequency of the brush head. Again, various methods can be used for the detection, such as contrast-based or blur-based detection.

Other sensors can be also used to determine the tissue contact such as a separate proximity sensor such as a capacitive sensor, or a force sensor such as a Hall sensor. These options are all intended to be represented by the sensor 26 in Figure 1.

Another option is the use of localization information provided by the brush itself by using the IMU 24. It is known to use different sensors such as the IMU 24 to determine location of a powered toothbrush within the oral cavity. The localization information can be then used to derive which oral tissues are in the vicinity (or opposite) the brush head such that they are contacted by the camera.

Another option, for a powered device, is to monitor the drive train current to detect changes (such as damping of the amplitude, phase changes or delays) due to the tissue contact. This monitoring thus functions as load sensing of the drive train of the device.

Figure 2 shows a toothbrush head 14 comprising a platen 30 having a front side 32 from which bristles 33 extend. The camera 18 has two image collection surfaces 34 on a back side 36 of the platen. The image collection surface or surfaces may instead be on a lateral side of the platen.

Thus, the image collection is of a region away from a treatment area. For tooth brushing, this region is less likely to have the images obscured by toothpaste foam.

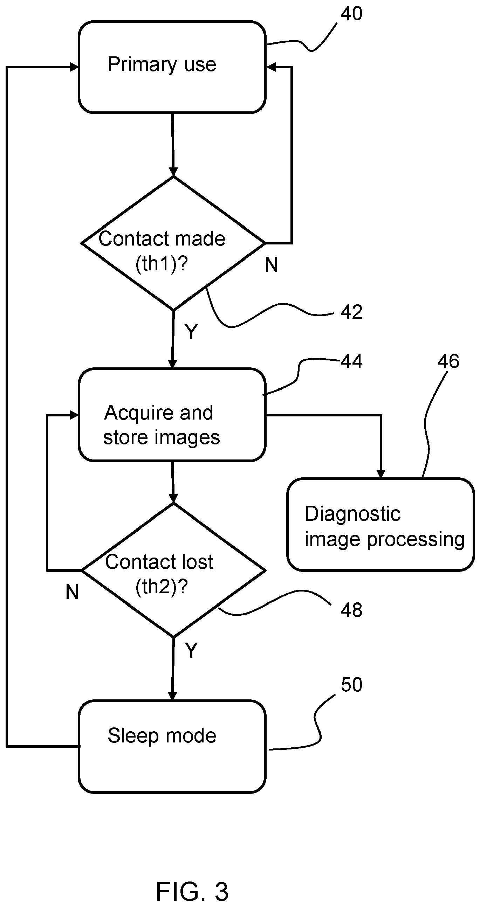

Figure 3 shows a method of capturing images of an internal surface of the oral cavity using the device described above.

In step 40, the device is being used for its primary purpose, e.g. tooth brushing in the case of a toothbrush.

In step 42, contact is detected of the image collection surface with oral tissue to be imaged. This detection for example makes use of a first threshold th1. As explained above, this may make use of image analysis, with images already being captured. Figure 3 however instead shows an example based on contact sensing by a separate alternative method, so that the camera can be shut down when there is no contact. This may provide a lower power solution that the use of image analysis to detect contact.

Images are then captured and stored in step 44 for further analysis.

The further analysis is shown as step 46. As mentioned above, it may take place in real time by the device itself (the processor of the toothbrush) or it may take place offline by another device to which the images are communicated. The further analysis may be used by the user, for example the device may provide a diagnosis and for example recommend a visit to a doctor/dentist, or the further analysis may be used by a medical professional.

In step 48 it is detected when contact is lost. This makes use of a second threshold th2, lower than the first threshold. Thus, images continue to be captured until the contact quality drops below a lower threshold than used to initiate the image storage.

In step 50, the camera is put into a sleep mode when contact has been lost (because the images are not needed for the contact sensing in this example).

The examples above relate to tooth/gum hygiene (toothbrushes, flossing systems, irrigators). The invention may instead be applied to a baby bottle teat or a pacifier teat.

The baby periodically actively engages with the teat during feeding or while sucking on a pacifier.

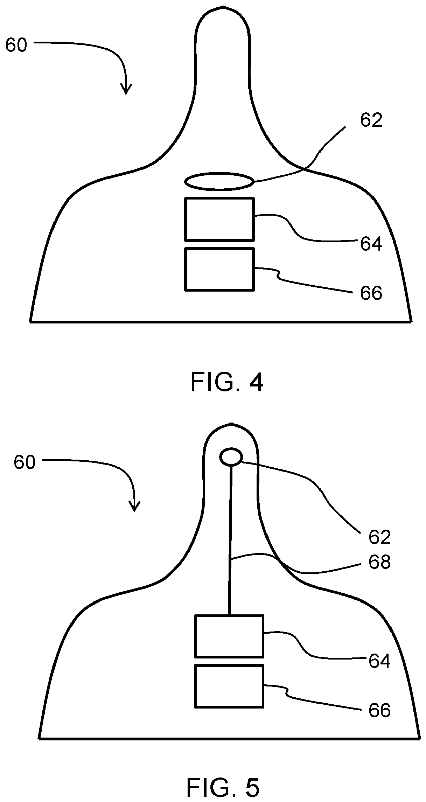

Figure 4 shows a teat 60 which may be the outlet for a milk bottle or it may be a soothing teat of a pacifier (basically in the form of a teat with no outlet).

Figure 4 shows a lens 62 forming the image collection surface, the camera hardware 64 and a power supply and controller unit 66. The image collection surface is around the base of the teat, for imaging the lips when the teat is in use.

Figure 5 shows a different position for the lens 62 and again also shows the camera hardware 64 and the power supply and controller unit 66. The image collection surface is near the tip of the teat, for imaging the tongue when the teat is in use. An optical fiber 68 couples the light receiving lens to the camera hardware.

The teat 60 can be sterilized or cleaned after use by providing the camera module as a click in unit which is applied to and removed from the teat.

The image processing of the captured and stored images for example involves spectral analysis to differentiate between a white milk tongue coating and an oral thrush tongue coating, and thereby enable a differential diagnosis to be provided to the user.

The baby bottle teat or pacifier teat may be used during feeding/soothing, or it may be used as a separate accessory e.g. after feeding is over, to diagnose any other oral problems such as anatomical defects (fissured (inner) lips, palate abnormalities or malformations) or sucking strength analysis etc.

There are various possible diagnostic functions enabled by the system. It may for example be used for tongue surface health imaging (detecting a coating, discoloration, lesions, or a breach in continuity (fissures) etc.,) and differential diagnosis of common pathologies.

The image reconstruction of tongue or other tissue features uses only images in focus, to reconstruct the 2D or 3D tongue morphology. The images may be analyzed to select toothpaste and saliva free images.

The image analysis may be used for diagnosis of benign and malignant oral lesions (oral mucosa, red spots, bubbles, herpes). The use of longitudinal data enables early detection.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner (optional)

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A device (10,60) for use in the mouth, for implementing a primary function, wherein the device comprises:
a body (14);
an optical imaging system (18) mounted at the body for capturing images of oral tissue in contact with the optical imaging system, wherein the optical imaging system has an image collection surface (34) and the capturing of images comprises a secondary function of the device; and
a processor (20) for controlling the collection and/or storage of images by the optical imaging system,
wherein the device is configured to detect contact of the image collection surface with oral tissue to be imaged, and
wherein the processor (20) is configured to store one or more images during said contact for further analysis or transmission.

2. The device of claim 1, wherein the optical imaging system comprises a fixed focus camera (18) and/or a contact microscope, for contact imaging.

3. The device of claim 1 or 2, comprising an apparatus for implementing the primary function to a first oral tissue, and wherein the optical imaging system is for implementing the secondary function to a different second oral tissue.

4. The device of any one of claims 1 to 3, wherein the processor (20) is configured to:
apply a first contact threshold (th1) to detect when contact is made and to store the one or more images in response to said contact; and optionally
apply a second contact threshold (th2) to cease storing images.

5. The device of any one of claims 1 to 4, wherein the processor is configured to detect the contact based on detection of an image focus of images and/or reflected light intensity level captured by the optical imaging system (18).

6. The device of any one of claims 1 to 5, wherein the device comprises a driven portion, and:
the processor (18) is configured to detect the contact based on analysis of an image movement of images captured by the optical imaging system (18); or.
the device further comprises a load sensor for sensing a drive load of the driven portion, and the processor (18) is configured to detect the contact from the sensed drive load.

7. The device of any one of claims 1 to 5, wherein the device comprises a force sensor for sensing a force applied to the device, and the processor is configured to detect the contact from the sensed force.

8. The device of any one of claims 1 to 5, comprising a proximity sensor, wherein the processor (20) is configured to the detect contact from an output of the proximity sensor.

9. The device of any one of claims 1 to 5, comprising a location sensing system, wherein the processor (20) is configured to detect the contact from an output of the location sensing system.

10. The device of any one of claims 1 to 9, comprising an oral care or treatment device, and wherein the primary function is to clean or treat oral tissue.

11. The device of claim 10, comprising an oral cleaning device.

12. The device of claim 11, comprising a platen (30) having a front side (32) from which oral cleaning elements (34) extend, wherein the image collection surface (34) is on a back side (36) or lateral side of the platen (30).

13. The device of any one of claims 1 to 9, comprising:
a baby pacifier, wherein the primary function is to provide a simulated nipple to pacify a baby; or
a teat (60) for a baby feeding bottle, wherein the primary function is to provide a simulated nipple for baby feeding.

14. A method of capturing images of an internal surface of the oral cavity using a device which is for implementing a primary function and comprises a body, and an optical imaging system with an image collection surface, mounted at the body for capturing images of oral tissue in contact with the optical imaging system, the method comprising:
(42) detecting contact of the image collection surface with oral tissue to be imaged; and
(44) storing one or more images during said contact for further analysis or transmission,
wherein the capturing of images comprises a secondary function of the device.

15. The method of claim 14, comprising applying a first contact threshold to detect when contact is made and to store the one or more images in response to said contact, and optionally applying a second contact threshold to cease storing images.
